# EUROPEAN PATENT APPLICATION

(11) **EP 2 194 475 A1**
(43) Date of publication of application: **09.06.2010**
(21) Application number: 10003190.5
(22) Date of filing: 03.01.2006
(51) Int. Cl.: G06F 19/00, A61B 5/00

(54) **Method and system for the management of data for a patient-controlled insulin therapy**

(30) Priority: 04.01.2005 IT BO20050002
(62) Divisional of application: 06000055.1
(71) Applicant: Lifescan, Inc., California CA 95035 (US)
(72) Inventor: The designation of the inventor has not yet been filed
(74) Representative: Carpmaels & Ransford

(57) **Abstract**

The present method for the interactive management of data concerning a patient-controlled insulin therapy for a diabetic patient requires the use of a cellular phone for interacting with the patient and with his doctor, by means of a computer program stored and run on the same cellular phone, and working on a database containing all the necessary data structures.

The aforesaid method comprises: the periodic input of data concerning the current patient's glycemic level, and their storage in a dedicated memory of said program database; the periodic input and storage of data concerning the physical activity performed by the patient; the periodical input and storage of data concerning the timing and dosing of insulin self-administrated by the patient; the input and storage of data concerning exceptional events for the patient's health; the input and storage of a base insulin dosing prescribed by the patient's doctor.

The managing method moreover comprises a processing phase for defining, depending on the above data relating to the glycemic level, influence of said exceptional health events, and base insulin dosing, a patient's daily-alimentary budget, based on information relating to a plurality of dishes stored in the program database. The alimentary budget comprises dishes containing a total amount of carbohydrates suitable to keep the patient's glycemic level within an optimal range of values. The processing phase also computes an updated base insulin dosing, depending on the course of the stored glycemic level and on the aforesaid computed alimentary budget.

A significant set of acquired and stored data, concerning a pre-defined period of time, is then sent to the patient's doctor, by means of a SMS, MMS or E-Mail message, so as to allow him to prescribe an updated base insulin dosing.

## Description

The present invention fits into that technical sector concerning the therapeutic treatment of diabetic patients in a patient-controlled regimen.

More particularly the present invention relates to a method for the interactive management of information concerning a therapy fit to keep the patient's glycemic level in an optimal range, compatibly with their pathologic state. The present invention moreover refers to a system for carrying out the aforesaid method.

It is known that patients suffering from diabetes must be periodically subjected to measurements of their glycemic level, with daily frequency or even more, and for different physical and metabolic activity. This is essential to the doctor in order to establish a precise therapeutic program for each patient and, if necessary, in order to modify said program.

The aforesaid program comprises both the administration of suitably computed doses of insulin and a definition of the amount of carbohydrates that can be consumed by the patient each day. The insulin doses, as well as the amount of carbohydrates, depend on several factors, even variable for the same patient as, by example, the physical activity that it carries out during a whole day.

Normally there is the need of a strict relation between the patient and his doctor. The first one must learn to follow several behaviour rules stated by the second one. These rules comprise the self-controlling frequency of the glycemic level, defining a daily diet which allows a right amount of carbohydrates to be assimilated, and carrying out a constant and programmed physical activity. Moreover the patient must periodically inform his doctor the course of the glycaemia levels which have been self-detected by the same patient, and supply also him with information relating the physical activity he has carried out, together with some possible alimentary excess. Then the doctor, according to the aforesaid data, confirms the insulin dosing, or alternately he defines a new insulin dosing.

In order to apply the aforesaid rules correctly, a diabetic patient must normally maintain a sort of diary, wherein he duly notes down all the data that he has taken each day. Nevertheless, the aforesaid operation is often not properly carried out, as the patient could forget to note down some data which could appear not important at all, or he could be annoyed by the complex and long calculations about the carbohydrates contents of his meal. This generally leads the patient to decide to not compile his diary, or to compile it partially.

The diabetic patient also tends to be in difficulties when, by example, he must (or he wants to) substitute a specific course of his alimentary program with another one, whose carbohydrates content is not known to him. In this case he is not able to define the correct portion to eat in order to keep the normal insulin dosing, or in order to modify said dosing should he overeat.

The afore described situation, which in any case fall in the average behaviour of the diabetic patients, often lead the same patients to have a non-optimal, and however undesirably variable, glycemic level.

It is an object of the present invention to propose a method, and a system for carrying it out, for managing of personal data involved in the therapy of a diabetic patient, said method being fit to provide the patient with an interactive instrument able to simplify both all the data relating to his pathology, and the patient's interaction with his doctor.

Another object of the present invention is to propose a method and a system which can provide the patient, in real time, with all the suggestions which are helpful to adjust and optimise his glycemic level.

A further object of the present invention is to provide the diabetic patient with a management system as explained above, which is moreover usable by the patient itself with devices that are of common, daily use, and which doesn't oblige him to learn the use of more complex, dedicated devices.

All the aforementioned objects are fully attained, according to the Claims contents, by a method for interactively managing of data relating to a patient-controlled insulin therapy for a diabetic patient, comprising: the periodic acquisition of data relating to the patient's glycemic level, and storing said data in a dedicated area of a database of a portable computing device; the periodic acquisition of data relating to the physical activity performed by said patient, and storing said data in a dedicated area of said database; the periodic acquisition of data relating to times and dosing of insulin administration to said patient, and storing said data in a dedicated area of said database; the acquisition of data relating to exceptional events for the patient's health and storing said data in a dedicated area of said database; storing, in said database, of information relating to a base insulin dosing for said patient, prescribed by the doctor thereof; computing said data relating to glycemic level, times and mode of performing physical activities, influence of said exceptional events and insulin dosing for defining, for said patient, a daily alimentary budget, based on information concerning a plurality of dishes stored in said database, said alimentary budget having an amount of carbohydrates suitable for keeping the patient's glycemic level within an optimal value range, and for defining a new current insulin dosing, depending on the course of the stored glycemic level and on said alimentary budget; periodic transmission of a data set comprised in said acquired and stored data to the patient's doctor, said data being significant for a definition of a new base insulin dosing.

All the characteristic features of the present invention, as they will result from the appended Claims, are pointed out in the following detailed description, with reference to the attached drawing tables, wherein:
- figure 1 shows a main block diagram of a user computer program which carries out the method according to the present invention;
- figure 2 shows a block diagram of a procedure of the computer program of figure 1, indicated therein with identification number 1;
- figure 3 shows a block diagram of a procedure of the computer program of figure 1, indicated therein with identification number 2;
- figure 4 shows a block diagram of a procedure of the computer program of figure 1, indicated therein with identification number 3;
- figure 5 shows a block diagram of a procedure of the computer program of figure 1, indicated therein with identification number 4;
- figure 6 shows a block diagram of a procedure of the computer program of figure 1, indicated therein with identification number 5;
- figure 7 shows a block diagram of a procedure of the computer program of figure 1, indicated therein with identification number 6;
- figure 8 shows a block diagram of a procedure of the computer program of figure 1, indicated therein with identification number 7;
- figure 9 shows a block diagram of a procedure of the computer program of figure 1, for defining the adjustment of the total insulin dose.

With reference to the aforesaid figures, there is illustrated a preferred embodiment of a method for interactively managing of data relating to a patient-controlled insulin therapy for a diabetic patient, carried out according to the present invention.

This method is preferably carried out by a system comprising a portable computing device, more preferably a cellular phone, and an user application program, fit to run on the aforesaid cellular phone. This latter is, of course, of the kind provided with a permanent read/write solid state memory, able to store user programs and data and to load and interactively run said user programs, that is displaying on its display a set of running options provided by said programs, inputting the user's choices and subsequently displaying the corresponding results.

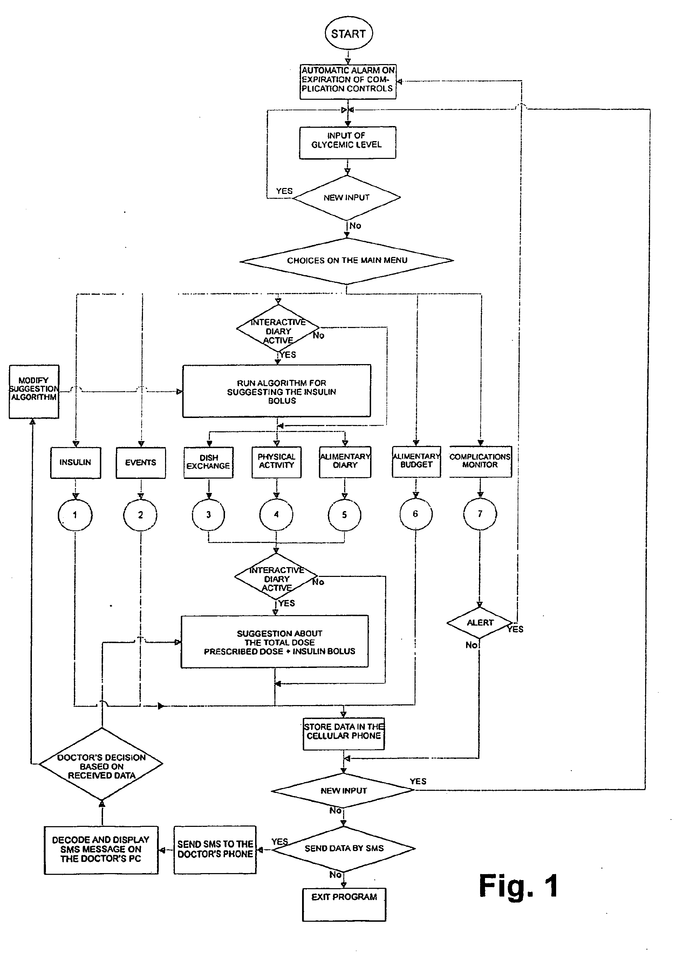

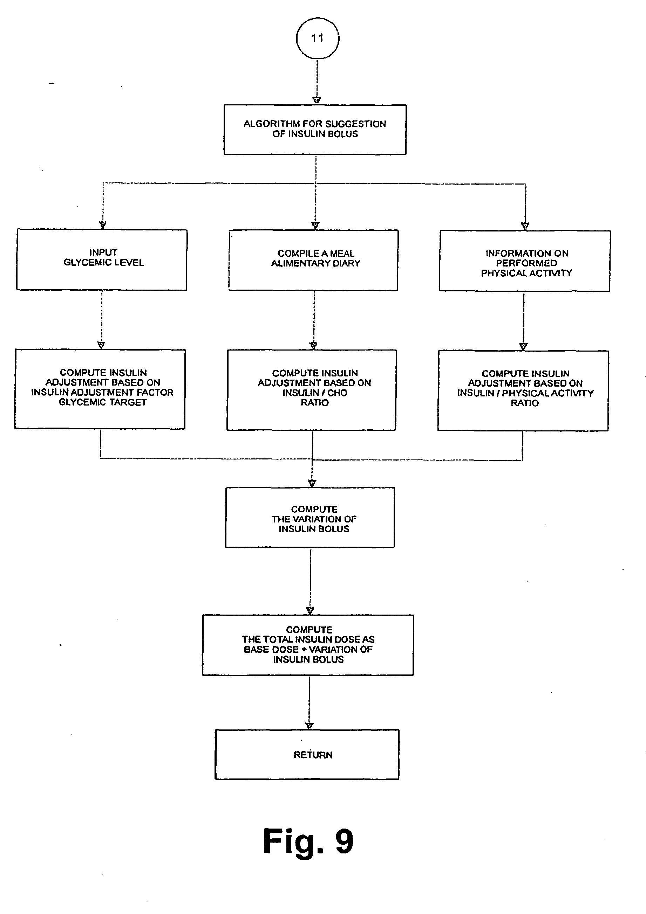

The aforesaid system moreover comprise a user program, fit to run on the above described cellular phone and substantially designed according to the block diagrams shown in the figures 1 to 9. The user program can be run by the patient at every moment, according to running modes which are known and substantially depending on the used model of cellular phone.

More particularly, the user program comprises a user interaction procedure, which is activated at the program start-up, which allows the patient to display several program options and to select one or more among them, by means of a user interface whose aspect substantially depends on the operating system running on the used cellular phone. By consequence, the details about the user interface design and the programming sequences and languages which allow to obtain the method steps and functions that will be described in the following are not strictly relating to the present invention, and therefore they will not be further investigated.

The user interaction procedure (see figure 1) first of all comprises a request to the patient for inserting of a control value of the patient's glycemic level, which has been taken by means of a portable glucometer, and for storing the acquired glycemic level value. In fact, this mandatory request acts as a reminder for the patient, as a periodical, constant control of the glycemic level is of fundamental importance for the whole therapeutic program.

Therefore, a reminder to the patient for inputting the above parameter is displayed each time he starts up the user program, even if the program is run for using the other program functions that will be better defined later. The glycemic level can be inputted just typing its value on the cellular phone keyboard.

A variant of the aforesaid procedure uses a substantially automated input procedure for the glycemic level value. In this case both the glucometer and the cellular phone are provided with standard communication lines and protocols, allowing them to dialog and transfer the detected glycemic level value. These communication lines and protocols are normally present and available on several cellular phone and portable glucometer models. The most common and easily usable communication lines comprise the infra-red (IrDA) and "Bluetooth" standards. Both of them use wireless communication channels and are particularly simple and easy to use.

The interaction procedure then continues providing the patient a choice among a plurality of headings from a main menu, each heading corresponding to a function of the management system. A given procedure corresponds to each of said headings.

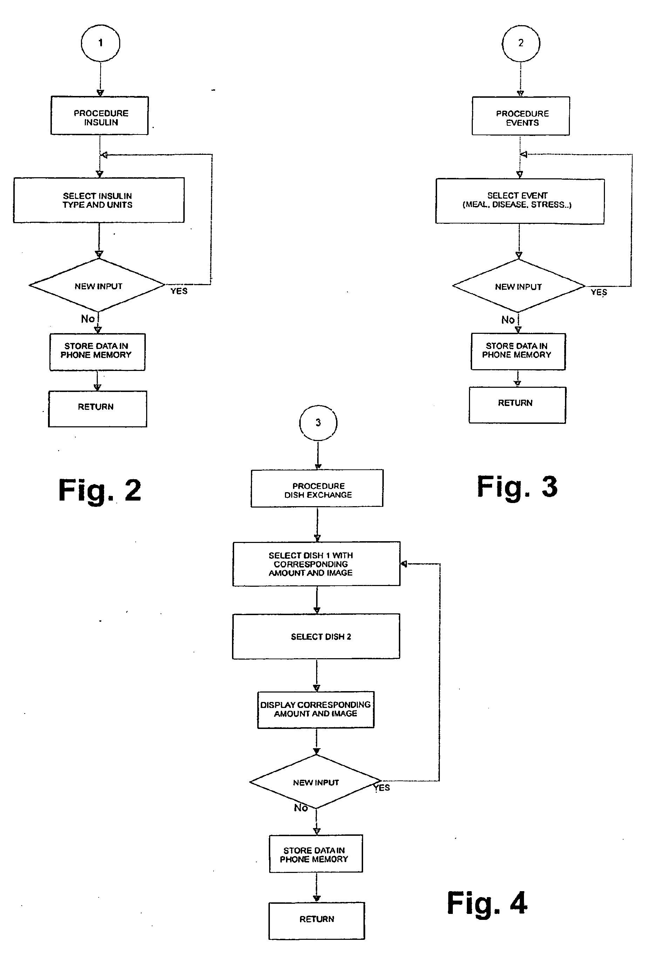

A first procedure, indicated with numeral 1 in figure 1 and shown with a block diagram in figure 2, relates to the input of dose and type of insulin that the patient administers by himself, and is called by the same patient after he has completed the self-administration. Once the corresponding data have been input, the same data are stored in a memory area suitably arranged for this purpose in the program database.

A second procedure, indicated with numeral 2 in figure 1 and shown with a block diagram in figure 3, relates to storing of exceptional events that could have occurred to the patient, and which could affect the definition of the subsequent therapeutic program. More particularly, such events could comprise data relating a disease, special stressing situations, extra meals which are out of the normal alimentary program, etc. These data are duly input and stored in their permanent memory area on the cellular phone.

A third procedure, indicated with numeral 3 in figure 1 and shown in figure 4, carries out a very important operating phase of the method according to the present invention, which relates to the food exchange. In order to make this procedure working, there are stored in a database in the phone memory a set of information relating to a plurality of dishes, which are present in the patient's diet, with the specific carbohydrates content for each of them, and more particularly with a graphic representation thereof. Said graphic representation preferably consists of a series of digital pictures, each one representing a dish with a different amount of the given food, corresponding to a different portion that can be consumed by the patient.

When activated by the patient, the above procedure asks for selecting a dish which is present in the program database, and an amount of the selected dish. Then the procedure asks the patient for selecting another dish for substituting the original one, among those present in the program database. The food exchange procedure then displays a picture of the substituting dish which shows a portion of the substituting dish that is equivalent, in terms of carbohydrates content, to the substituted portion of food.

The above described operation is particularly helpful in the daily management of the patient's diet. This latter, in fact, becomes free to modify in real time the composition of his meal on the base of the dishes that are available at that moment. This is particularly helpful when the patient is going to have a meal at a restaurant or at another place where one or more dishes of his daily diet planning is missing.

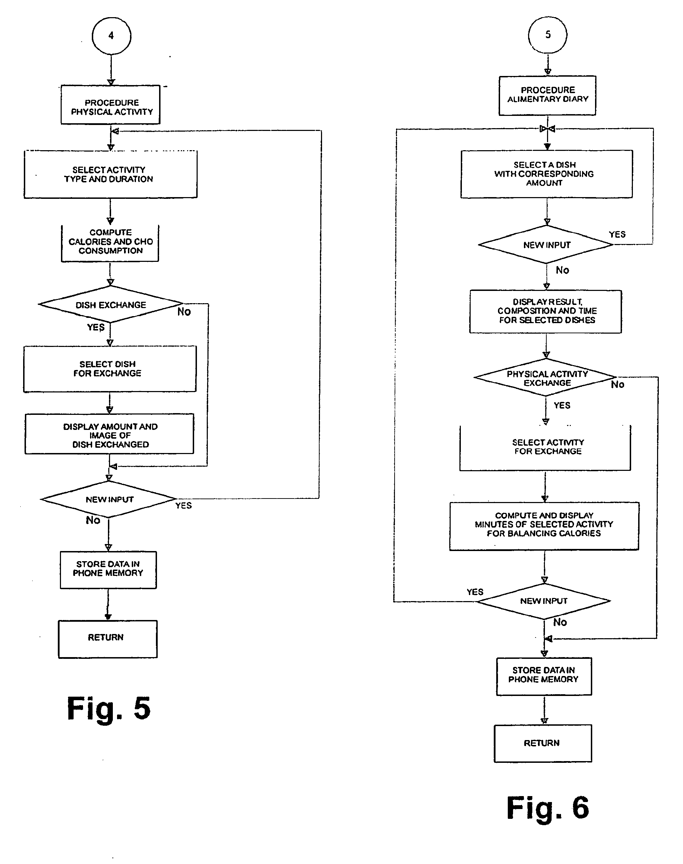

A fourth procedure, indicated with numeral 4 in figure 1 and shown in figure 5, relates to storing and handling of information regarding the diabetic patient's physical activity. Once started, the procedure 4 asks the patient for input the kind of physical activity he carried out, for its level of intensity and its duration. Starting from those information the procedure computes and stores in the program database, according to commonly used conversion parameters, the amount of the consumed calories and/or of carbohydrates corresponding to the physical activity that has been carried out by the patient. These data, for the patient's convenience, can be also displayed in the form of picture of a particular dish, selected by the same patient among those present in the food database, the picture representing the amount of that dish which compensates, in terms of calories and/or of carbohydrates, the calories or carbohydrates used by the patient during his physical activity. This graphical information is normally very helpful for the patient which intends to vary his diet, and which intends to "buy" some more food "paying" it with an improvement of his normal physical activity. The aforesaid data could be also used by the management program for carrying out some further step of the present method, as it will be described in the following.

A fifth procedure, indicated with the numeral 5 in figure 1 and shown in figure 6, relates the management of an alimentary diary, fit to define with the patient a composition of the daily dishes which would be compatible with the total amount of carbohydrates allowed by the patient's diet. The patient is asked for inserting the desired dishes and their corresponding amounts. The result in terms of a total amount in carbohydrates is then displayed, and the patient is asked again to change the dishes composition. He can also "buy" some additional food, over the scheduled amount, and "pay" it performing an amount of physical activity sufficient to burn a corresponding amount of calories. For this purpose, once the patient has selected a preferred sport, the procedure automatically computes its duration and intensity. Data representing the selected dishes and the physical activity are then stored in the program database.

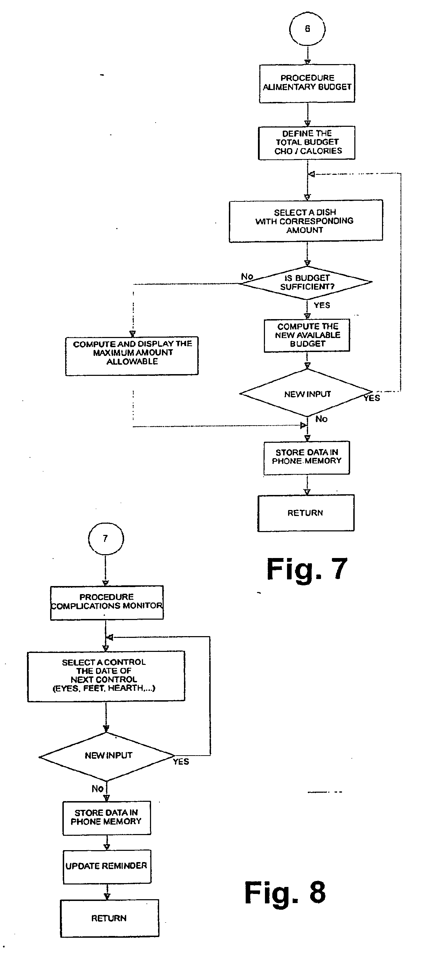

A sixth procedure, indicated with numeral 6 in figure 1 and shown in figure 7, handles an alternate managing mode for the patient's meal organisation, according to a "alimentary budget" mode. When started, said procedure asks the patient for defining a total budget in terms of calories and/or amount of carbohydrates available in his diet. Subsequently the patient is asked for select a dish which is present in the program database, together with the desired amount of said dish. The selected portion is then displayed as a picture, wherein the selected amount of food is represented on a dish, in order to give the patient an exact idea of how big the selected portion is. The selected dish content in terms of calories and/or carbohydrates is then deducted from the available alimentary budget. The above step is then repeated until the alimentary budget is set to zero. The data concerning the selected meal are then stored in the computer database.

A seventh procedure, indicated with numeral 7 in figure 1 and shown in figure 8, is fit to handle a reminder for the controls that the patient has to carry out in order to prevent the common complications that could arise because of his pathology (typically, eyes problems, blood circulation in arms and legs, heart problems etc.). The reminder procedure comprises a section that is started up by the patient by means of the interaction procedure, which allows the patient to input data concerning the expiration dates of the controls to be made for the different complications, and to store them in the program database. It moreover comprises a section, which is periodically and automatically started up by the user program, wherein said expiration dates are checked and compared with the current date. In case of at least one of the expiration dates is approaching, the procedure sends a visual and/or acoustic alert to the patient, using the cellular phone resources.

For each patient's choice comprising a definition of a meal composition and the computing of debit calories (compilation of the alimentary diary) or credit calories (performing of physical activity), the user program moreover starts up a procedure for suggesting the correct insulin bolus to the patient, which computes and suggests an insulin bolus based on the above parameters. In fact, the suggested value is computed, by means of an algorithm which implements evaluating modes substantially known and normally applied manually by a doctor, on the base of a series of additional parameters. Said parameters first comprise a base insulin dosing, prescribed to the patient by his doctor and previously stored in the program database.

There are moreover comprised the current patient's glycemic level, his feeding, whose data have been stored in the program database by the above described alimentary diary and alimentary budget procedures, and the performed physical activity, whose data have been stored by the corresponding physical activity procedure.

Starting from the above parameters, the insulin bolus suggestion procedure computes a variation of the same insulin bolus, and defines the total patient's insulin dose as a sum of the base insulin dose plus the computed insulin bolus variation.

For each operation carried out by the above described procedures, the user program moreover asks the patient for sending the significant stored data to his doctor. If the patient intends to inform the doctor about his situation, all the significant data are coded and sent via SMS, MMS or by means of a E-Mail message (if said operation is allowed by the used cellular phone model and by the patient's provider) to a phone number or E-Mail address provided by the same doctor, and previously stored in the program database.

A decoding procedure, belonging to a complementary user program running on the doctor's personal computer, decodes and arranges the received data, and makes them available to the doctor. This latter can then analyse them, and eventually define a new base insulin dosing for that patient. Data relating said new base insulin dosing can then sent back to the patient, by means of a voice telephone call, via E-Mail or by a suitably coded SMS or MMS message, directly to the user program running on the patient's cellular phone.

By the way, if he deems it necessary, at this moment the doctor could define a new algorithm for suggesting the insulin bolus, and send it to the user program running on the patient's cellular phone, for an automatic update.

The above described user program could, obviously, feature further setup, maintenance and on-line guide functions, which could be helpful for an optimal program working, and which are typical of each complex application program. Neither the structure nor detailed programming of this functions really relate to the present invention, and therefore they will not be explained in further detail herein.

Several advantages can be obtained by the use of the method and system according to the present invention. First of all, the patient is provided with a complete, versatile and intuitive instrument for controlling all the significant parameters of his pathology, for storing all the data that must be acquired and for obtaining the information necessary to carry out the routine operations relating his behaviour and therapeutic treatment.

A further advantage arises from the help, also immediate and intuitive, that he gets for calculating his accurate alimentary needs and his accurate insulin dosing, according to his normal daily activities, or for varying in real time said dosing and amounts depending on new and sudden demands, as well as on his alimentary wishes.

Yet another great advantage which is achieved with the present invention is that the aforesaid organising instrument is given to the patient on a hardware support, i.e. his cellular phone, that is highly familiar to him, that he always carries with him and that is of easy use. In this way the patient will be induced to exploit even its accessory features, as the present managing system is, without being forced to buy, learn and carry with him a new, special device.

It has to be intended that the above specification has been described for exemplifying and non limiting purposes. Therefore, all the modifications and variants of the present invention have to be considered included in the present technical solution, as it results from the above description and from the appended Claims.

## Claims

1. System for interactive managing of data relating to a patient-controlled insulin therapy, the system comprising:
a cellular phone, provided with a permanent read/write memory for storing user data, and fit to run interactive user programs;
a database, residing in said permanent memory of said cellular phone;
a user program stored in said cellular phone memory, the user program containing instructions for:
(a) allowing a patient to select and run a set of different program options;
(b) acquiring a glycemic level value, which can be run by said interaction procedure and fit to input and store in said program database the current glycemic level value for said patient;
(c) inputting and storing data relating to the physical activity performed by said patient, which inputting and storing are on demand of the aforesaid patient;
(d) inputting and storing data relating to exceptional events occurred to the patient and concerning the patient's health;
(e) processing said data concerning:
(i) glycemic level, duration and intensity of the physical activity performed by the patient,
(ii) influence of said exceptional events relating to the patient's health, and
(iii) data representing a base insulin dosing prescribed to said patient;
(f) defining for the same patient a daily alimentary budget, said definition based on information residing in said program database, said database including carbohydrates contents of a plurality of dishes and digital images of said dishes, each image representing a plate containing portions of said dishes having a carbohydrate content substantially equal to the corresponding stored value, wherein said daily alimentary budget comprises an amount of carbohydrate suitable for keeping the patient's glycemic level within an optimal range, and
(g) defining a new current insulin dosing, depending on the course of the stored glycemic value and of said alimentary budget; and
(h) periodically and automatically sending, via said cellular phone, all significant data to the patient's doctor, suitable for defining an updated base insulin dosing for said patient.

2. System according to claim 1, said user program further comprising instructions for:
(a) building a meal starting from a predefined amount of carbohydrates,
(b) allowing the patient to subsequently select portions from a set of stored dishes, and
(c) correspondingly deducting the carbohydrates content of the selected portion of each dish from the predefined amount of carbohydrates, while some is available.

3. System according to claim 2, said user program further comprising instructions for allowing the patient to exchange, on demand, a dish of said meal with a portion of a substitute dish, among those stored in the program database, which is equivalent to the substituted one in terms of carbohydrates content.

4. System according to claim 2, said user program further comprising a mechanism for computing the calories consumed by a given physical activity performed by the patient, and the corresponding update of the patient's daily alimentary budget.

5. System according to claim 1, further comprising receiving and storing in said cellular phone, said updated base insulin dosing from the patient's doctor.

6. System according to claim 1 or claim 5, wherein data is sent to or received from the patient's doctor by means of SMS, MMS or E-Mail messages.

7. System according to claim 1 or claim 5, further comprising a complementary user program, embodied on a doctor's personal computer, said complementary computer program operative to receive, decode and display said messages sent by the cellular phone, and to send to the same phone a message containing the updated base insulin dosing.

8. System according to claim 9, said user program further comprising instructions for enabling input and storage of data relating to expiration dates for controls to be made by the patient in relation to the diabetes-related complications, and sending visual and/or acoustic signals to the patient when said expiration dates are approaching.

9. System according to claim 9, wherein said cellular phone is in communication with a portable glucometer, and is configured to read one or more values of said glycemic level.

10. System according to claim 9, wherein said standard communication line is a wireless infrared (IrDA) communication line.

11. System according to claim 19, wherein said standard communication line is a wireless communication line.

12. A method for interactive managing data relating to a patient-controlled insulin therapy for a diabetic patient, comprising:
periodically acquiring the patient's glycemic level, and storing it in a dedicated area of a database in permanent memory of a cellular phone;
acquiring on demand of the patient data relating to physical activity performed by said patient, and storing said data in a dedicated area of said database;
periodically acquiring data relating to times and dosing of insulin administration to said patient, and storing said data in a dedicated area of said database;
acquiring data relating to exceptional events occurred to the patient and storing said data in a dedicated area of said database;
storing, in said database, information relating to a base insulin dosing for said patient, prescribed by the patient's doctor;
computing:
(i) glycemic level, duration and intensity of the physical activity performed by the patient,
(ii) influence of said exceptional events relating to the patient's health, and
(iii) data representing a base insulin dosing prescribed to said patient;
defining for the same patient a daily alimentary budget, said definition based on information residing in said database, said database including carbohydrates contents of a plurality of dishes and digital images of said dishes, each image representing a plate containing portions of said dishes having a carbohydrate content substantially equal to the corresponding stored value, wherein said daily alimentary budget comprises an amount of carbohydrate suitable for keeping the patient's glycemic level within an optimal range, and
defining a new current insulin dosing, depending on the course of the stored glycemic value and of said alimentary budget; and
periodically and automatically sending, via said cellular phone, all significant data to the patient's doctor, suitable for defining an updated base insulin dosing for said patient.

13. Method according to claim 12, wherein said daily alimentary budget is modified, on demand of said patient, by substituting one or more alternate dishes, whose characteristics in term of carbohydrate contents are stored in said database, the amount of said alternate dishes being defined so as to contain the same amount of carbohydrates as the substituted dish.

14. Method according to claim 12, wherein said exceptional health events comprise diseases, conditions of particular stress and hypoglycemia events.

15. Method according to claim 12, further comprising receiving and storing in said cellular phone, said updated base insulin dosing from the patient's doctor.

16. Method according to claim 12, wherein data is sent to or received from the patient's doctor by means of SMS, MMS or E-mail messages.

17. Method according to claim 16, **characterized in that** said periodic acquisition of a current value of the patient's glycemic level is carried out by said cellular phone which is in communication with a portable glucometer and is configured to read one or more values of said glycemic level.
